# FASCICULE DE BREVET EUROPEEN

(11) **EP 2 991 572 B1**
(45) Date de publication et mention de la délivrance du brevet: **11.01.2017**
(21) Numéro de dépôt: 14722253.3
(22) Date de dépôt: 02.04.2014
(51) Int. Cl.: A61B 17/80

(54) **DISPOSITIF D'OSTÉOTOMIE, EN PARTICULIER POUR LA RÉALISATION DU SCARF EXTRÊME DANS LE TRAITEMENT DE L'HALLUX VALGUS SÉVÈRE.**
OSTEOTOMIEVORRICHTUNG, INSBESONDERE ZUR DURCHFÜHRUNG EINER EXTREMEN SCARF-OSTEOTOMIE BEI DER BEHANDLUNG VON SCHWEREM HALLUX VALGUS
OSTEOTOMY DEVICE, IN PARTICULAR FOR PERFORMING EXTREME SCARF OSTEOTOMY IN THE TREATMENT OF SEVERE HALLUX VALGUS

(30) Priorité: 29.04.2013 FR 1353917
(43) Date de publication de la demande: 09.03.2016
(73) Titulaire: BIOTECH ORTHO, 13300 Salon-de-Provence (FR)
(72) Inventeur: LEEMRIJSE, Thibaut, B-1200 Bruxelles (BE); MAESTRO, Michel, F-06200 Nice (FR); DEVOS, Bernhard, B-9831 Deurle (BE); RELAVE, Marc, F-42160 Andrezieu Boutheon (FR); BESSE, Jean-Luc, F-69970 Chaponnay (FR)
(74) Mandataire: Weber, Etienne Nicolas
(86) Numéro de dépôt international: PCT/FR2014/050781
(87) Numéro de publication internationale: WO 2014/177783

(56) Documents cités:
- WO-A1-2012/112642
- US-A1- 2010 256 687

## Description

La présente invention concerne un dispositif d'ostéotomie, en particulier pour la réalisation de l'ostéotomie de SCARF bien connue des praticiens, et notamment pour la réalisation du SCARF extrême (de grand déplacement) dans le traitement de l'Hallux Valgus sévère.

Toutefois, ce dispositif est tout à fait indiqué dans la réalisation de SCARF modérés, ou encore pour la fixation de l'ostéotomie du premier rayon sur des SCARF dits extrêmes lors de simples révisions chirurgicales, ou par exemple pour des patients ostéoporotiques pour lesquels seul le cortex (la corticale) est solide (voire anormalement rigide).

L'Hallux Valgus est une déformation du pied très répandue correspondant à la déviation du premier métatarsien et du gros orteil du premier rayon. L'angle inter-métatarsien IM (entre les métatarses du premier et du deuxième rayons) et l'angle Hallux Valgus HV (entre le métatarse du premier rayon et le gros orteil) sont des mesures radiographiques couramment utilisées pour évaluer le degré de difformité du métatarse du premier rayon. Ces mesures ont permis d'établir la classification suivante : un Hallux Valgus est considéré comme :
- normal si : HV < 15° et IM < 9°
- moyen si : 15° < HV < 20° et 9° < IM < 11°
- modéré si : 20° < HV < 40° et 11° < IM < 16°
- sévère si : HV > 40° et IM > 16°

Il existe plusieurs techniques pour corriger ces difformités, l'intervention se faisant toujours sur le premier métatarsien :
- l'ostéotomie de la tête du premier métatarse (ostéotomie de Reverdin Isham) qui permet de réorienter la surface articulaire du métatarse et corrige ainsi la déformation ;
- opération de Keller correspondant à une arthroplastie par résection de la base de la première phalange du gros orteil, qui reste utilisée en cas de dégénérescence arthrosique de la première articulation métatarso-phalangienne ;
- arthrodèse ou « fusion » métatarso-phalangienne du gros orteil.

Les techniques les plus courantes restent cependant :
- l'ostéotomie diaphysaire telle que l'ostéotomie de SCARF ;
- l'ostéotomie basale telles que l'ostéotomie en chevron ou l'ostéotomie d'ouverture avec ou sans comblement de greffon.

Il est possible d'associer à ces interventions sur le premier métatarse, la correction de la première phalange, par exemple par l'ostéotomie d'Akin qui corrige le valgus sur la première phalange.

Toutes ces techniques offrent de bons résultats à la fois cliniques, et radiographiques. Toutefois, certaines complications peuvent intervenir telles que des risques de thrombose, de récidive d'Hallux Valgus, un effet « tuile » (encastrement des deux fragments osseux), ou encore des métatarsalgies (douleurs de l'avant-pied, en regard des métatarsiens).

Les différentes études réalisées dans ce domaine indiquent que les ostéotomies de SCARF fournissent une méthode efficace pour le traitement de l'Hallux Valgus sévère. Toutefois, les dispositifs actuels ne sont pas indiqués pour les corrections des Hallux Valgus sévères par la technique du SCARF extrême.

En effet, on connaît par exemple des plaques d'ostéotomie présentant une première partie destinée à être appliquée et fixée sur la corticale de l'os à traiter, et une seconde partie destinée à être enfouie en centromédullaire, dans la partie diaphysaire de l'os à traiter.

Par exemple, le document WO-2012/112642 décrit une plaque orthopédique présentant une première extrémité comportant un trou de vis de blocage destiné à recevoir une vis de blocage et, à distance de ce trou de blocage, un logement de compression qui s'étend à partir de la face de la plaque en regard de l'os et qui reçoit une vis de compression dont l'axe forme un angle compris entre 10° et 70° avec l'axe longitudinal de la plaque. Cette plaque comporte encore une deuxième extrémité munie d'un chanfrein assurant l'insertion de ladite plaque dans l'os.

Cette plaque est particulièrement indiquée pour les corrections de l'Hallux Valgus par la technique du CHEVRON. Elle n'est pas adaptée pour la mise en oeuvre de la technique du SCARF, et encore moins du SCARF extrême pour le traitement de l'Hallux Valgus sévère.

De plus, la présence du logement pour la vis de compression qui s'étend à partir de la face inférieure (destinée à être appliquée en regard de l'os à traiter), rend le dispositif très complexe à utiliser.

On connait encore le document US-2009/0036931 qui décrit une plaque de chirurgie orthopédique pour l'ostéosynthèse de fragments d'os du pied, cette plaque comprenant une partie proximale destinée à prendre appui et être fixée sur la face externe du premier fragment d'os, cette partie proximale comprenant au moins une ouverture de passage d'une vis orthopédique, et une partie distale destinée à être placée dans le logement centromédullaire du deuxième fragment d'os, cette partie distale présentant également au moins une ouverture de passage d'une vis orthopédique.

Cette plaque est particulièrement indiquée pour la réalisation de l'ostéotomie sous-capitale, ou encore pour la réalisation de l'arthrodèse du Lapidus.

De plus, sa mise en oeuvre, notamment par la nécessité de visser des vis orthopédiques dans la partie distale destinée à être placée dans la partie centromédullaire du fragment osseux nécessite une instrumentation particulière, et un temps de mise en oeuvre long et fastidieux.

Bien que cette plaque ne présente pas de logement protubérant gênant, là encore elle n'est pas adaptée pour la mise en oeuvre de la technique du SCARF, et encore moins du SCARF extrême pour le traitement de l'Hallux Valgus sévère.

En outre, chacune des plaques décrites dans les documents WO-2012/112642 et US-2009/0036931 comporte une lame destinée à être insérée dans la partie centromédullaire de l'os à traiter. Cette lame de forme générale plate est encombrante, elle est difficile à faire rentrer dans le spongieux de la partie centromédullaire, et nécessite une instrumentation spécifique adaptée à sa forme.

De plus, son extrémité est légèrement aiguisée pour pouvoir creuser son emplacement dans la partie centromédullaire au risque d'abimer la corticale interne au niveau de ladite partie centromédullaire.

Ces lames rigides ne sont pas modelables, ou du moins, elles ne le sont pas dans toutes les directions.

Un but de la présente invention est de proposer aux chirurgiens praticiens, un dispositif d'ostéotomie de déplacement extrême, notamment pour la réalisation du SCARF extrême dans le traitement de l'Hallux Valgus sévère, exempt des inconvénients susmentionnés.

Selon l'invention, ce but est atteint grâce à un dispositif d'ostéotomie de déplacement extrême remarquable en ce qu'il comprend :
- une plaque d'ostéosynthèse de fixation destinée à être verrouillée sur la corticale de l'os à traiter, constituée d'une première partie proximale apte à être fixée sur la corticale interne de la diaphyse métatarsienne de l'os, d'une partie distale apte à être fixée sur la corticale externe, à proximité de l'épiphyse du métatarse, et d'une partie centrale reliant lesdites parties proximale et distale, chacune des parties proximale et distale de la plaque étant munie d'au moins un trou pour le passage de vis orthopédiques ;
- un système de tenue centromédullaire proximal constitué par une queue à pointe mousse s'étendant depuis l'extrémité de la partie proximale de la plaque.

Selon un mode de réalisation avantageux, la partie distale de la plaque d'ostéosynthèse comprend deux trous pour le passage de vis orthopédiques verrouillées dont les axes sont perpendiculaires, ou approximativement perpendiculaires au plan de la plaque, et la partie proximale de la plaque comprend un trou pour le passage d'une vis orthopédique verrouillée dont l'axe est perpendiculaire, ou approximativement perpendiculaire au plan de la plaque et un trou pour le passage d'une vis orthopédique non verrouillée dont l'axe est angulé par rapport à l'orthogonale au plan de la plaque.

Selon un mode de réalisation avantageux, le dispositif selon l'invention comprend des vis verrouillées à tête filetée qui peut être auto-taraudeuse et les pourtours des trous de verrouillage pour les passages des vis orthopédiques verrouillées sont exécutés dans un matériau présentant des propriétés mécaniques autorisant un auto-taraudage par les têtes filetées des vis verrouillées.

Selon un mode de réalisation avantageux, les pourtours des trous de verrouillage pour le passage des vis orthopédiques verrouillées sont en polymères de la famille des polyaryléthercétones (PAEK), par exemple en polyétheréthercétone (PEEK).

Selon un mode d'exécution, les pourtours des trous pour le passage des vis orthopédiques verrouillées sont constitués par des inserts en polymères de la famille des polyaryléthercétones (PAEK), par exemple en polyétheréthercétone (PEEK).

Selon un mode de mise en oeuvre, l'axe du trou de passage de la vis orthopédique non verrouillée disposé dans la partie proximale de la plaque présente une angulation comprise entre 0° et 45°, et préférentiellement 20°.

Selon un mode de mise en oeuvre avantageux, la queue centromédullaire est inclinée par rapport au plan dans lequel est comprise la plaque, par exemple d'un angle compris entre 0° et 30°, et préférentiellement 10°.

Suivant une autre disposition caractéristique, la queue est de section polygonale, par exemple de section carrée dont les angles sont arrondis.

Selon un mode de réalisation avantageux, la face inférieure de la plaque destinée à être en contact avec l'os est granuleuse (corrindonée) afin d'assurer une meilleure accroche osseuse.

Selon un mode de réalisation, la plaque et la queue du dispositif d'ostéotomie sont exécutées en tout matériau biocompatible présentant la robustesse nécessaire, par exemple en titane pur.

Le dispositif d'ostéotomie selon l'invention procure plusieurs avantages intéressants. Notamment :
- de permettre le renforcement du montage de base autobloquant du SCARF, du fait que peu d'efforts sont appliqués sur ledit dispositif ;
- d'être adaptable à toutes situations du fait que la queue centromédullaire peut être légèrement pliée par rapport à la plaque, de sorte que le dispositif d'ostéotomie selon l'invention est facilement conformable à la morphologie de l'os à traiter, et ceci dans toutes les directions, à la fois par sa forme et son matériau ;
- de permettre un bon plaquage de la plaque du dispositif d'ostéotomie sur la surface de l'os du fait de l'utilisation de vis verrouillées ;
- de permettre l'ajout d'une compression supplémentaire lors du serrage de la vis non verrouillée (ou de rappel) afin de rapprocher les fragments osseux ;
- de permettre l'ajout d'une rigidité supplémentaire et un maintien accru grâce à la queue proximale positionnée en centromédullaire et prenant appui sur la corticale interne de l'os ;
- de permettre de ne pas abimer la surface de la corticale interne grâce à la présence de la pointe mousse à l'extrémité de la queue.

De plus, ce dispositif est très fiable à l'usage, sa mise en place est aisée, il répond donc parfaitement aux attentes des chirurgiens en termes de facilité de mise en place et de fiabilité à l'usage.

Les buts, caractéristiques et avantages ci-dessus, et d'autres encore, ressortiront mieux de la description détaillée qui suit et des dessins annexés dans lesquels :

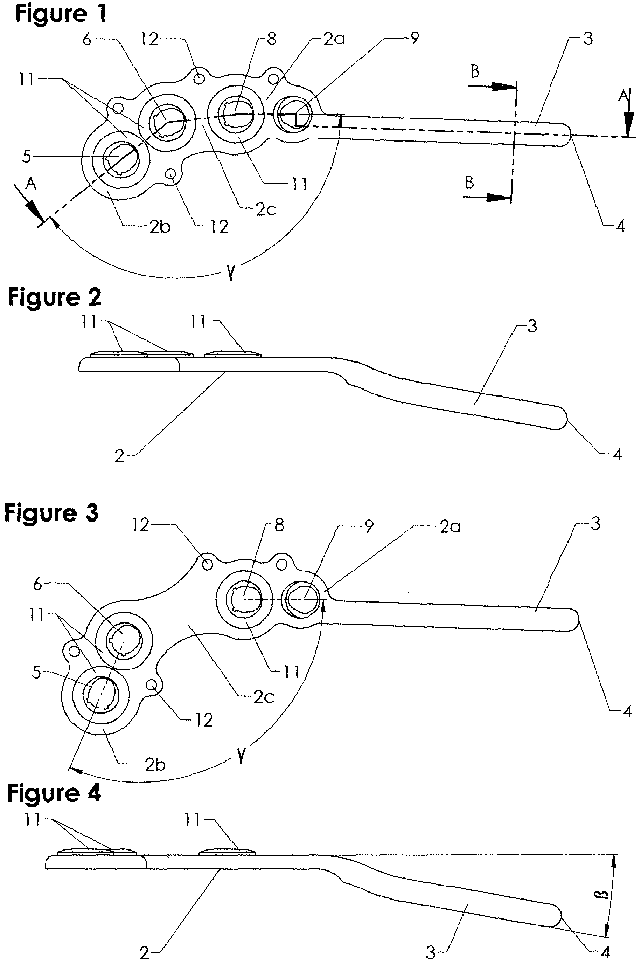

La figure 1 est une vue en plan d'un premier exemple de réalisation du dispositif d'ostéotomie selon l'invention, dit « de petite dimension » pour une utilisation sur le pied droit.
La figure 2 est une vue de côté du dispositif d'ostéotomie selon la figure 1.
La figure 3 est une vue en plan d'un deuxième exemple de réalisation du dispositif d'ostéotomie selon l'invention, dit « de grande dimension » pour une utilisation sur le pied droit.
La figure 4 est une vue de côté du dispositif d'ostéotomie selon la figure 3.

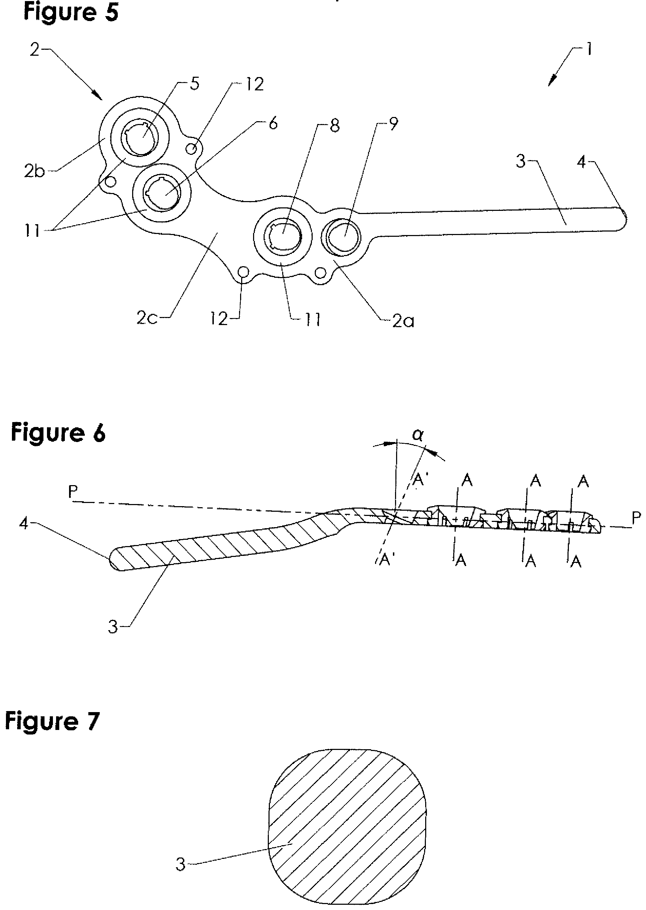

La figure 5 est une vue en plan d'un troisième exemple de réalisation du dispositif d'ostéotomie selon l'invention, dit « de grande dimension » pour une utilisation sur le pied gauche.
La figure 6 est une vue en coupe selon la ligne A-A de la figure 1 du dispositif d'ostéotomie selon l'invention.
La figure 7 est une vue en coupe selon la ligne B-B de la figure 1, du dispositif selon l'invention.

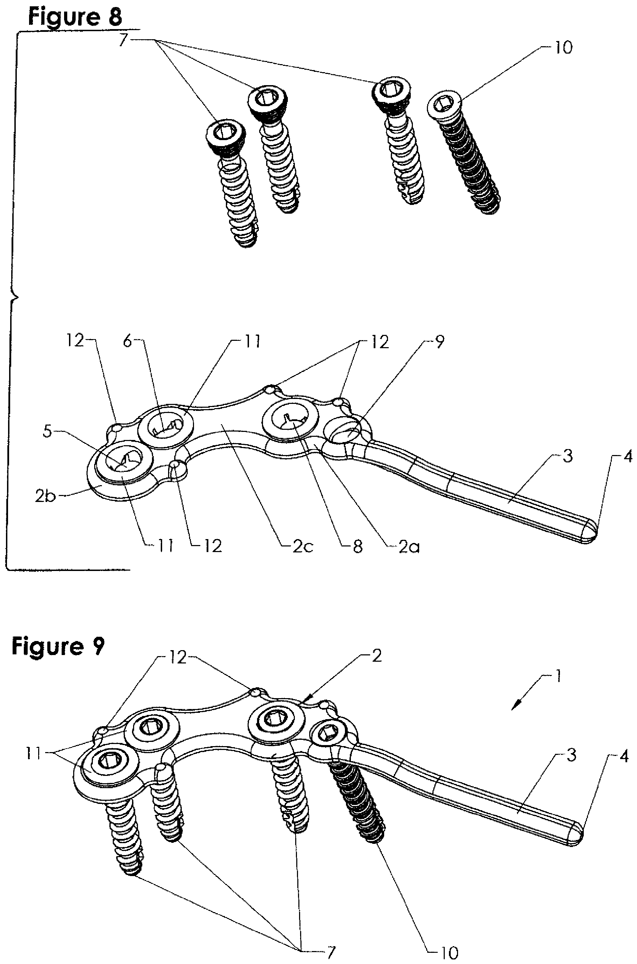

La figure 8 est une vue en perspective éclatée du dispositif et de vis orthopédiques utilisables pour la fixation dudit dispositif.
La figure 9 est une vue analogue à la figure 8 et montrant les vis orthopédiques en place dans le dispositif selon l'invention.

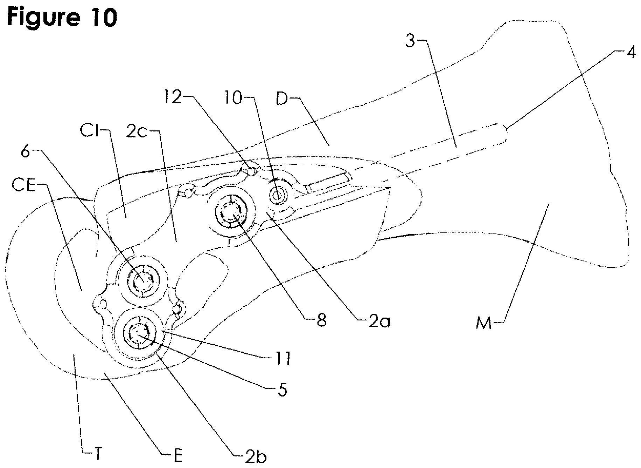

La figure 10 est une vue en perspective illustrant le dispositif selon l'invention en place sur un métatarse.

On se réfère auxdits dessins pour décrire des exemples intéressants quoique nullement limitatifs, de réalisation d'un dispositif d'ostéotomie de déplacement extrême selon l'invention.

Dans le présent exposé et dans les revendications, les mots « proximale » et « distale » sont utilisés en référence au positionnement fonctionnel des éléments constitutifs du dispositif revendiqué, la partie proximale étant la plus proche du talon du pied tandis que la partie distale est la plus proche des orteils du pied.

Egalement, dans le présent exposé et dans les revendications, la face interne de la corticale (CI) correspond à l'interface entre l'os spongieux contenu dans la partie diaphysaire et l'os cortical.

Le dispositif d'ostéotomie 1 selon l'invention comprend une plaque d'ostéosynthèse et des vis orthopédiques de verrouillage à tête filetée qui peut être auto-taraudeuse et une vis orthopédique non verrouillée.

Plus précisément, le dispositif d'ostéotomie 1 selon l'invention comprend :
- une plaque d'ostéosynthèse 2 de fixation apte à être verrouillée sur la corticale de l'os à traiter (à savoir le premier métatarse M) à l'aide de vis orthopédiques,
- un système de tenue centromédullaire proximal constitué par une queue 3 à pointe mousse 4 disposée dans le prolongement de la partie proximale de la plaque.

La plaque de fixation 2 est avantageusement constituée :
- d'une partie proximale 2a apte à être fixée sur la corticale interne de la diaphyse D métatarsienne de l'os,
- d'une partie distale 2b apte à être fixée sur la corticale externe, à proximité de l'épiphyse E du métatarse,
- et d'une partie centrale 2c reliant lesdites parties proximale et distale.

La plaque 2 présente une forme générale incurvée, par exemple la partie proximale de la plaque et la partie distale de la plaque forment un angle Y compris entre 90° et 180°. Elle présente une longueur par exemple comprise entre 20 mm et 30 mm.

Il peut être proposé des dispositifs de petite dimension (par exemple pour l'utilisation sur un métatarse de petite taille), comportant une plaque de l'ordre de 23 mm de longueur, et des dispositifs de grande dimension (par exemple pour l'utilisation sur un métatarse de grande taille), comportant une plaque de l'ordre de 26 mm. Dans le cas des dispositifs dits « de grande dimension », la surface de la partie centrale 2c est plus importante.

Par exemple, pour des dispositifs dits « de petite dimension », Y est de l'ordre de 141° tandis que pour des dispositifs dits « de grande dimension », Y est de l'ordre de 113°.

Elle peut encore présenter une largeur de l'ordre de 7 mm et une épaisseur de l'ordre de 1,2 mm.

Chacune des parties proximale 2a et distale 2b de la plaque 2 est munie d'au moins un trou pour le passage de vis orthopédiques.

Selon l'exemple illustré, chacune des parties proximale 2a et distale 2b de la plaque 2 est munie de deux trous pour le passage de vis.

De préférence, la partie distale 2b comprend deux trous 5 et 6 pour le passage de deux vis verrouillées 7. Les axes A-A des trous 5 et 6 sont perpendiculaires, ou approximativement perpendiculaires au plan P-P de la plaque 2, et la partie proximale 2a comprend un trou 8 pour le passage d'une vis verrouillée 7 dont l'axe A-A est perpendiculaire, ou approximativement perpendiculaire au plan P-P de la plaque 2 et un trou 9 pour le passage d'une vis non verrouillée 10 dont l'axe A'-A' est incliné d'un angle α par rapport à l'orthogonale au plan P-P de la plaque 2.

De manière connue en soi, les vis verrouillées 7 comportent une tête filetée de forme conique qui peut être auto-taraudeuse. Ces vis présentent, par exemple, un diamètre de l'ordre de 2,40 mm en distal.

La vis non verrouillée 10 (encore appelée vis de rappel) connue en soi présente, par exemple, un diamètre de l'ordre de 2,30 mm en distal.

De manière avantageuse, l'axe A'-A' du trou de passage de la vis non verrouillée dont est munie la partie proximale 2a de la plaque 2 présente une angulation α comprise entre 0° et 45°, et préférentiellement 20°.

Avantageusement, la queue 3 est inclinée d'un angle β par rapport à la plaque 2. Par exemple cet angle β est compris entre 0° et 30°, et préférentiellement, il est de l'ordre de 10°.

Cette queue présente une longueur de l'ordre de 20 mm, de sorte que les dispositifs dits « de petite taille » présentent une longueur totale de l'ordre de 43 mm et que les dispositifs dits « de grande taille » présentent une longueur totale de l'ordre de 46 mm.

La queue 3 présente avantageusement une section polygonale, par exemple et de manière préférée, elle est de section carrée à angles arrondis, d'environ 2 mm de côté, par exemple.

De préférence et avantageusement, la face inférieure de la plaque 2 destinée à être en contact avec l'os est granuleuse (corrindonée) afin d'assurer une meilleure accroche osseuse. Cette accroche osseuse permet à la plaque de ne pas glisser lors de la pose sur l'os à traiter et assure ainsi un positionnement aisé de ladite plaque. L'aspect granuleux permet, une fois la plaque mise en place, une meilleure ostéointégration.

Selon le mode d'exécution représenté, les pourtours 11 des trous de verrouillage 5, 6 et 8 pour les passages des vis verrouillées 7 sont exécutés dans un matériau présentant des propriétés mécaniques autorisant un auto-taraudage par les têtes filetées des vis verrouillées.

Selon un mode de réalisation avantageux, les pourtours 11 des trous de verrouillage 5, 6 et 8 pour le passage des vis verrouillées 7 sont en polymères de la famille des polyaryléthercétones (PAEK), par exemple en polyétheréthercétone (PEEK).

Selon l'exemple représenté, les pourtours 11 des trous de verrouillage 5, 6 et 8 pour le passage des vis verrouillées 7 sont constitués par des inserts en polymères de la famille des polyaryléthercétones (PAEK), par exemple en polyétheréthercétone (PEEK).

Le verrouillage des têtes de vis 7 dans le PEEK est inspiré du système décrit dans le brevet FR-2.845.588 « Dispositif d'ostéosynthèse autobloquant », au nom de Biotech International et commercialisé sous la marque déposée « EASYLOCK ».

La tête auto-taraudeuse des vis verrouillées 7 creuse son propre sillon hélicoïdal récepteur dans le pourtour 11 des trous de verrouillage 5, 6 ou 8 dans lesquels elles sont engagées, de sorte que lesdites vis se trouvent ensuite automatiquement bloquées dans la plaque lorsque leur tête se trouve serrée dans son logement.

D'autre part, cette caractéristique autorise une angulation sélective des vis verrouillées 7, par rapport à l'axe A-A desdits trous de verrouillage de la plaque, en fonction des besoins, de + ou - 10°.

La plaque 2 comporte encore plusieurs orifices 12 pour le passage de broches de fixation permettant le maintien de la plaque pendant sa mise en place.

Selon un mode de réalisation, la plaque et la queue du dispositif d'ostéotomie sont exécutées en tout matériau biocompatible présentant la robustesse nécessaire, par exemple elles sont réalisées en titane pur pour une biocompatibilité maximale. De plus, cela permet de plier légèrement la queue 3 par rapport à la plaque d'ostéosynthèse 2 (ou inversement), et ceci dans toutes les directions, de sorte à rendre le dispositif d'ostéotomie conformable à la morphologie de l'os à traiter.

Le dispositif selon l'invention peut donc se décliner en version de petite taille, de l'ordre de 43 mm de longueur, ou en version de grande taille, de l'ordre de 46 mm de longueur, en version « droite » pour une utilisation sur pied droit, et en version « gauche » pour une utilisation sur pied gauche.

La mise en place du dispositif d'ostéotomie selon l'invention par la méthode du SCARF (bien connue des praticiens) consistant à obtenir un montage autobloquant comporte les étapes suivantes :
1. réaliser la résection de la tête T latérale externe du métatarse M ;
2. réaliser les différentes coupes selon la technique du SCARF de manière connue en soi ;
3. choisir le dispositif d'ostéotomie 1 à utiliser en fonction de la morphologie des os et de la surface disponible (petit ou grand dispositif, gauche ou droit) ;
4. positionner le dispositif d'ostéotomie 1 en commençant par faire rentrer la queue 3 de tenue centromédullaire dans la partie diaphysaire D de la partie proximale du SCARF et, si nécessaire, modeler légèrement ladite queue 3 (ou la plaque 2) de sorte que la partie proximale 2a de la plaque 2 soit appuyée sur la face interne de la corticale CI, et que la partie distale 2b de la plaque 2 soit appuyée sur la face externe de la corticale CE dudit métatarse ;
5. maintenir la plaque dans cette position à l'aide de broches B passées au travers des trous 12 ;
6. utiliser, de manière connue en soi, un guide pour préparer le forage et veiller à ne pas abimer les inserts en PEEK ainsi que le trou angulé non verrouillé et forer lesdits emplacements des vis verrouillées et de la vis non verrouillée ;
7. utiliser, de manière connue en soi, une jauge de profondeur afin de connaître la longueur appropriée des vis à utiliser ;
8. positionner la vis non verrouillée 10 et commencer son vissage sans la visser entièrement ;
9. positionner les trois vis verrouillées 7 et les visser en commençant par le trou distal 5 ;
10. terminer de visser la vis non verrouillée 10.

## Revendications

1. Dispositif d'ostéotomie (1) en particulier pour la réalisation du SCARF extrême dans le traitement de l'Hallux Valgus sévère, **caractérisé en ce qu'**il comprend:
- une plaque (2) de fixation apte à être verrouillée sur la corticale de l'os à traiter (M), constituée d'une première partie proximale (2a) apte à être fixée sur la corticale interne (CI) de la diaphyse (D) métatarsienne de l'os, d'une partie distale (2b) apte à être fixée sur la corticale externe (CE), à proximité de l'épiphyse (E) du métatarse, et d'une partie centrale (2c) reliant lesdites parties proximale et distale, chacune des parties proximale et distale de la plaque étant munie d'au moins un trou (5, 6 ; 8, 9) pour le passage de vis orthopédiques (7, 10) ;
- un système de tenue centromédullaire proximal constitué par une queue (3) à pointe mousse (4) s'étendant depuis l'extrémité de la partie proximale (2a) de la plaque (2).

2. Dispositif d'ostéotomie (1) selon la revendication 1, **caractérisé en ce que** la partie distale (2b) de la plaque (2) comprend deux trous (5, 6) pour le passage de vis orthopédiques verrouillées (7) dont les axes (A-A) sont perpendiculaires, ou approximativement perpendiculaires au plan (P-P) de la plaque, et la partie proximale (2a) de la plaque (2) comprend un trou (8) pour le passage d'une vis orthopédique verrouillées (7) dont l'axe (A-A) est perpendiculaire, ou approximativement perpendiculaire au plan (P-P) de la plaque et un trou (9) pour le passage d'une vis orthopédique non verrouillée (10) dont l'axe (A'-A') est angulé par rapport à l'orthogonale au plan (P-P) de la plaque.

3. Dispositif d'ostéotomie (1) suivant l'une quelconque des revendications 1 ou 2, **caractérisé en ce que** les pourtours (11) des trous de verrouillage (5, 6 et 8) pour les passages des vis orthopédiques verrouillées (7) sont exécutés dans un matériau présentant des propriétés mécaniques autorisant un auto-taraudage par les têtes filetées desdites vis verrouillées.

4. Dispositif d'ostéotomie (1) selon la revendication 3, **caractérisé en ce qu'**il comprend des vis verrouillées à tête filetée et **en ce que** les pourtours (11) des trous de verrouillage (5, 6 et 8) pour le passage des vis orthopédiques verrouillées (7) sont en polymères de la famille des polyaryléthercétones (PAEK), par exemple en polyétheréthercétone (PEEK).

5. Dispositif d'ostéotomie (1) suivant la revendication 3, **caractérisé en ce qu'**il comprend des vis verrouillées à tête filetée et **en ce que** les pourtours (11) des trous de verrouillage (5, 6 et 8) pour le passage des vis orthopédiques verrouillées (7) sont constitués par des inserts en polymères de la famille des polyaryléthercétones (PAEK), par exemple en polyétheréthercétone (PEEK).

6. Dispositif d'ostéotomie (1) selon l'une quelconque des revendications 1 à 5, **caractérisé en ce que** l'axe (A'-A') du trou (9) de passage de la vis orthopédique non verrouillée (10) disposé dans la partie proximale (2a) de la plaque (2) présente une angulation (α) comprise entre 0° et 45°, et préférentiellement 20°.

7. Dispositif d'ostéotomie (1) suivant l'une quelconque des revendications 1 à 6, **caractérisé en ce que** la queue (3) est inclinée par rapport au plan (P-P) dans lequel est comprise la plaque (2), par exemple d'un angle (β) compris entre 0° et 30°, et préférentiellement 10°.

8. Dispositif d'ostéotomie (1) selon l'une quelconque des revendications 1 à 7, **caractérisé en ce que** la queue (3) est de section polygonale, par exemple de section carrée dont les angles sont arrondis.

9. Dispositif d'ostéotomie (1) suivant l'une quelconque des revendications 1 à 8, **caractérisé en ce que** la face inférieure de la plaque (2) destinée à être en contact avec l'os est granuleuse (corrindonée) afin d'assurer une meilleure accroche osseuse.

10. Dispositif d'ostéotomie (1) selon l'une quelconque des revendications 1 à 9, **caractérisé en ce que** la plaque (2) et la queue (3) du dispositif d'ostéotomie sont exécutées en tout matériau biocompatible présentant la robustesse nécessaire, par exemple en titane pur.

## Patentansprüche

1. Osteotomievorrichtung (1), insbesondere zur Durchführung einer extremen SCARF-Osteotomie bei der Behandlung von schwerem Hallux Valgus, **dadurch gekennzeichnet, dass** sie folgende Bestandteile aufweist:
- eine Befestigungsplatte (2), die an der Kortikalis des zu behandelnden Knochens (M) verriegelt werden kann, welche aus einem ersten proximalen Teil (2a), der an die interne Kortikalis (CI) der metatarsalen Diaphyse (D) des Knochens befestigt werden kann, einem distalen Teil (2b), der an der externen Kortikalis (CE) in der Nähe der Epiphyse (E) des Mittelfußknochens befestigt werden kann, und aus einem mittleren Teil (2c), der den proximalen und distalen Teil miteinander verbindet, besteht, wobei jeder der proximalen und distalen Teile der Platte mit mindestens einem Loch (5, 6, 8, 9) zur Durchführung der orthopädischen Schrauben (7, 10) versehen ist.
- ein proximales Markraumnagelsystem, bestehend aus einem Einsteckende (3) aus Schaumstoff (4), das aus dem Ende des proximalen Teils (2a) der Platte (2) herausragt.

2. Osteotomievorrichtung (1) nach Anspruch 1, dadurch gekenntzeichnet, dass der distale Teil (2b) der Platte (2) zwei Löcher (5, 6) zur Durchführung von orthopädischen Verriegelungsschrauben (7) aufweist, deren Achsen (A-A) senkrecht oder fast senkrecht zur Fläche (P-P) der Platte verlaufen, und dass der proximale Teil (2a) der Platte (2) ein Loch (8) zur Durchführung einer orthopädischen Verriegelungsschraube (7) aufweist, deren Achse (A-A) senkrecht oder fast senkrecht zur Fläche (P-P) der Platte verläuft sowie ein Loch (9) zur Durchführung einer nicht verriegelten orthopädischen Schraube (10), deren Achse (A'-A') in Bezug auf die Orthogonale zur Fläche (P-P) der Platte gewinkelt ist.

3. Osteotomievorrichtung (1) nach einem der Ansprüche 1 oder 2, **dadurch gekennzeichnet, dass** die Umfänge (11) der Verriegelungslöcher (5, 6 und 8) für die Durchführungen der orthopädische Verriegelungsschrauben (7) aus einem Werkstoff ausgeführt sind, der mechanische Eigenschaften aufweist, die ein automatisches Gewindebohren durch die Gewindeköpfe der Verriegelungsschrauben ermöglichen.

4. Osteotomievorrichtung (1) nach Anspruch 3, **dadurch gekennzeichnet, dass** sie Verriegelungsschrauben mit Gewindekopf aufweist und dass die Umfänge (11) der Verriegelungslöcher (5, 6 und 8) zur Durchführung der orthopädischen Verriegelungsschrauben (7) aus Polymeren der Klasse der Polyaryletherketone (PAEK), zum Beispiel Polyetheretherketon (PEEK), bestehen.

5. Osteotomievorrichtung (1) nach Anspruch 3, **dadurch gekennzeichnet, dass** sie Verriegelungsschrauben mit Gewindekopf aufweisen und dass die Umfänge (11) der Verriegelungslöcher (5, 6 und 8) zur Durchführung der orthopädischen Verriegelungsschrauben (7) aus Einsätzen aus Polymeren der Klasse der Polyaryletherketone (PAEK), zum Beispiel Polyetheretherketon (PEEK), bestehen.

6. Osteotomievorrichtung (1) nach einem der Ansprüche 1 bis 5, **dadurch gekennzeichnet, dass** die Achse (A'-A') des Lochs (9) zur Durchführung der orthopädischen nicht verriegelten Schraube (10), das im proximalen Teil (2a) der Platte (2) angeordnet ist, eine Winkeleinstellung (α) zwischen 0° und 45°, vorzugsweise 20° aufweist.

7. Osteotomievorrichtung (1) nach einem der Ansprüche 1 bis 6, **dadurch gekennzeichnet, dass** das Endstück (3) in Bezug auf die Fläche (P-P), in der die Platte (2) liegt, beispielsweise in einem Winkel (β) zwischen 0° und 30°, vorzugsweise 10°, geneigt ist.

8. Osteotomievorrichtung (1) nach einem der Ansprüche 1 bis 7, **dadurch gekennzeichnet, dass** das Endstück (3) einen polygonalen Querschnitt hat, beispielsweise einen quadratischen Querschnitt, dessen Ecken abgerundet sind.

9. Osteotomievorrichtung (1) nach einem der Ansprüche 1 bis 8, **dadurch gekennzeichnet, dass** die Innenfläche der Platte (2), die mit dem Knochen in Kontakt kommen soll, körnig (korundgestrahlt) ist, um eine bessere Knochenhaftung zu ermöglichen.

10. Osteotomievorrichtung (1) nach einem der Ansprüche 1 bis 9, **dadurch gekennzeichnet, dass** die Platte (2) und das Endstück (3) der Osteotomievorrichtung aus jedem biokompatiblen Material ausgeführt ist, das die nötige Robustheit aufweist, zum Beispiel aus reinem Titan.

## Claims

1. An osteotomy device (1), in particular for the Implementation of extreme SCARF in the treatment of severe Hallux Valgus, **characterised in that** it comprises:
- a fixing plate (2) capable of being locked on the cortical substance of the bone (M) to be treated, formed by a first proximal portion (2a) capable of being fixed on the internal cortical substance (CI) of the metatarsal diaphysis (D) of the bone, a distal portion (2b) capable of being fixed on the external cortical substance (CE) in the proximity of the epiphysis (E) of the metatarsus, and a central portion (2c) connecting said proximal and distal portions, each of the proximal and distal portions of the plate being provided with at least one hole (5, 6; 8, 9) for the passage of orthopaedic screws (7, 10); and
- a proximal centromedullary holding system formed by a shank (3) with a foam tip (4) extending from the end of the proximal portion (2a) of the plate (2).

2. An osteotomy device according to claim 1 **characterised In that** the distal portion (2b) of the plate (2) comprises two holes (5, 6) for passing locked orthopaedic screws (7) whose axes (A-A) are perpendicular or approximately perpendicular to the plane (P-P) of the plate, and the proximal portion (2a) of the plate (2) comprises a hole (8) for passing a locked orthopaedic screw (7) whose axis (A-A) is perpendicular or approximately perpendicular to the plane (P-P) of the plate and a hole for passing a non-locked orthopaedic screw (10) whose axis (A'-A') is angled with respect to the orthogonal to the plane (P-P) of the plate.

3. An osteotomy device (1) according to either one of claims 1 and 2 **characterised In that** the edges (11) of the locking holes (5, 6 and 8) for passing the locked orthopaedic screws (7) are made of a material having mechanical properties permitting self-tapping by the threaded heads of the locked screws.

4. An osteotomy device (1) according to claim 3 **characterised in that** it comprises locked screws with a threaded head and that the edges (11) of the locking holes (5, 6 and 8) for passing the locked orthopaedic screws (7) are made of polymers of the family of polyaryletherketones (PAEK), for example of polyetheretherketone (PEEK).

5. An osteotomy device (1) according to claim 3 **characterised in that** it comprises locked screws with a threaded head and that the edges (11) of the locking holes (5, 6 and 8) for passing the locked orthopaedic screws (7) are formed by inserts made of polymers of the family of polyaryletherketones (PAEK), for example of polyetheretherketone (PEEK).

6. An osteotomy device (1) according to any one of claims 1 to 5 **characterised In that** the axis (A'-A') of the hole (9) for passing the non-locked orthopaedic screw (10) disposed in the proximal portion (2a) of the plate (2) has an angulation (α) of between 0° and 45° and preferably 20°.

7. An osteotomy device (1) according to any one of claims 1 to 6 **characterised in that** the shank (3) is inclined with respect to the plane (P-P) in which the plate (2) Is disposed, for example at an angle (β) of between 0° and 30° and preferably 10°.

8. An osteotomy device (1) according to any one of claims 1 to 7 **characterised In that** the shank (3) is of a polygonal section, for example a square section with rounded angles.

9. An osteotomy device (1) according to any one of claims 1 to 8 **characterised in that** the lower face of the plate (2) that is Intended to be in contact with the bone is granular (corundum-coated) to ensure better bone adhesion.

10. An osteotomy device (1) according to any one of claims 1 to 9 **characterised in that** the plate (2) and the shank (3) of the osteotomy device are made of any biocompatible material of the necessary ruggedness, for example of pure titanium.
